# EUROPÄISCHE PATENTSCHRIFT

(11) **EP 0 858 600 B1**
(45) Veröffentlichungstag und Bekanntmachung des Hinweises auf die Patenterteilung: **12.12.2001**
(21) Anmeldenummer: 96934808.5
(22) Anmeldetag: 24.10.1996
(51) Int. Cl.: G01N 30/06, G01N 33/00

(54) **VERFAHREN UND VORRICHTUNG ZUR ANALYSE VON FLÜCHTIGEN SUBSTANZEN IN GASEN**
METHOD AND DEVICE FOR ANALYZING VOLATILE SUBSTANCES IN GASES
PROCEDE ET DISPOSITIF POUR ANALYSER DES SUBSTANCES VOLATILES DANS DES GAZ

(30) Priorität: 30.10.1995 LU 88675
(43) Veröffentlichungstag der Anmeldung: 19.08.1998
(73) Patentinhaber: EUROPÄISCHE ATOMGEMEINSCHAFT (EURATOM), 2920 Luxembourg (LU)
(72) Erfinder: SCHLITT, Helmut, I-21033 Cittiglio (IT)
(74) Vertreter: Weinmiller, Jürgen
(86) Internationale Anmeldenummer: EP9604624
(87) Internationale Veröffentlichungsnummer: WO9716723

(56) Entgegenhaltungen:
- INSTRUMENTATION SCIENCE & TECHNOLOGY, Bd. 22, Nr. 1, 1994, WAMPLER US, Seiten 25-38, XP002002837 A.H.J.GRÖMPIG ET AL.: "development of an automated, quasi-continuous method for the simultaneous determination of nitrogen oxides, aldehydes, and ketones in air."
- JOURNAL OF HIGH RESOLUTION CHROMATOGRAPHY, Bd. 8, Nr. 8, August 1985, HEIDELBERG DE, Seiten 411-414, XP002002838 H. FRANK: "quantitative gas chromatography using a new automated derivatizer"
- INTERNATIONAL LABORATORY (NEWS EDITION), Bd. 21, Nr. 3, April 1991, INC FAIRFIELD US, Seiten 38-40, XP002002839 E. GABRIELE: "automation of dissolution and injection in HPLC testing of lyophilized drugs"

## Beschreibung

Die Erfindung bezieht sich auf ein Verfahren und eine Vorrichtung zur automatischen Analyse von flüchtigen Substanzen in Gasen, insbesondere Luft, das nacheinander die folgenden Schritte enthält:
a) das zu analysierende Gas wird durch eine Absorptions- und ggf. Reaktionslösung geleitet,
b) die Lösung wird mittels einer Chromatographiemethode analysiert.

Solche Verfahren und Vorrichtungen sind bekannt. So ist in einem Aufsatz von A. Grömping und K. Cammann "Development of an automated, quasi-continuous method for the simultaneous determination of nitrogen oxides, aldehydes, and ketones in air", der in der Zeitschrift Instrumentation Science & Technology 22 (1994), Seiten 25 bis 38 veröffentlicht wurde, ein Verfahren der obigen Art beschrieben, bei dem das zu analysierende Gas durch ein mit einer Reaktions-lösung fast gefülltes Gefäß (Impinger) so geleitet wird, daß es durch die Lösung hochperlt. Nach der gewünschten Expositionszeit wird ein Teil der Lösung zur Analyse über Schlauchleitungen und eine peristaltische Pumpe in einen Chromatographen gepumpt, und der Rest der Lösung wird aus dem Gefäß entfernt. Das Gefäß wird dann gereinigt und mit frischer Lösung für den nächsten Analysezyklus beschickt. Alle Vorgänge werden automatisch über ein Ablaufprogramm überwacht und abgewickelt.

Ein wesentlicher Nachteil dieses Verfahrens liegt in der geringen Empfindlichkeit besonders aufgrund der beiden folgenden Effekte:
a) Zur Analyse mittels der beiden am häufigsten verwendeten chromatographischen Verfahren, d.h. der hochauflösenden Flüssigchromatographie (HPLC) und der Kapillar-Gaschromatographie (Kapillar-GC) können, ohne die erforderliche Trennleistung zu verlieren, nur etwa 20 µl bzw. 5 µl der Absorptions- oder Reaktionsflüssigkeit in die chromatographische Säule injiziert werden. Bei einem Volumen dieser Flüssigkeit von etwa 20 ml ist das nur 1% oder weniger des Probevolumens mit einem entsprechenden Verlust an Empfindlichkeit.
b) Das Absorptions- bzw. Reaktionsgefäß von mehreren ml kann mit vertretbarem Aufwand nicht so gereinigt werden, wie das für eine empfindliche Messung erforderlich wäre. Der "Memory-Effekt" ist also erheblich. Zum Memory-Effekt trägt auch die Leitung bei, durch die die zu analysierende Lösung nach der Exposition zum Chromatographen fließen muß. Auch diese Leitung muß also nach jeder Analyse gereinigt werden. Das Volumen des Gefäßes kann auch nicht beliebig verkleinert werden, da die Länge der erwähnten Leitung das Mindestvolumen des Gefäßes vorgibt. So sollen jeweils 20 ml Lösung zum Chromatographen gepumpt werden. Dabei wurden Grenzkonzentrationen von etwa 50 ppb Formaldehyd gemessen.

Die zunehmende Schadstoffbelastung von Innenräumen aufgrund verbesserter Raumisolation und der Verwendung von umweltschädlichen Klebern in der Möbelindustrie und für das Verlegen von Tapeten und Teppichböden hat das Bedürfnis nach einer preiswerten Messung und Überwachung auch geringer Schadstoffkonzentrationen, insbesondere der Konzentration von Formaldehyd im Konzentrationsbereich unterhalb 50 ppb geweckt.

Aufgabe der Erfindung ist es daher, ein neues Verfahren und eine daran angepaßte Vorrichtung anzugeben, mit denen es möglich ist, vollautomatisch eine Folge von Analysezyklen durchzuführen, die von der Entnahme einer Probe des zu analysierenden Gases über die Adsorption der Probe in einer Reaktionslösung bis zur Analyse dieser Lösung reichen und die Messung sehr geringer Schadstoffkonzentrationen erlauben, z.B. Formaldehyd in einer Konzentration von 1 µg/m³ oder ca. 0,8 ppb.

Diese Aufgabe wird erfindungsgemäß durch das Verfahren gelöst, das im Anspruch 1 definiert ist. Die Vorrichtung zur Durchführung dieses Verfahrens ist in Anspruch 3 gekennzeichnet. Bezüglich von Merkmalen einer bevorzugter Ausführungsform des Verfahrens wird auf Anspruch 2 verwiesen.

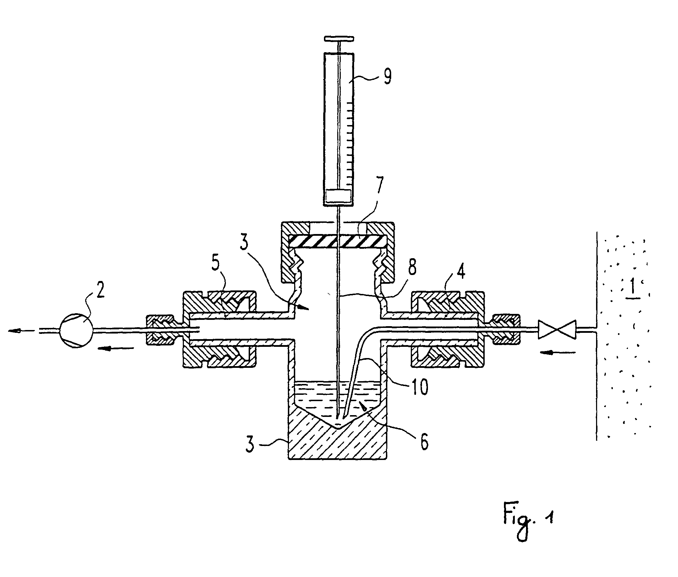

Die Erfindung wird nun anhand dieses bevorzugten Ausführungsbeispiels mit Hilfe der beiliegenden Zeichnung näher erläutert, die schematisch und im Schnitt ein Reaktionsgefäß zeigt, in dem eine Reaktionslösung dem zu analysierende Gas ausgesetzt wird.

Die Erfindung wird anhand des Formaldehydnachweises in der Raumluft erläutert, wenngleich sie auch auf den Nachweis anderer Stoffe, insbesondere Schadstoffe in der Luft oder in einem anderen Gas anwendbar ist.

Die zu analysierende Luft in einem Raum 1 wird mittels einer Saugpumpe 2 durch ein Reaktionsgefäß 3 gesaugt. Dieses Gefäß hat einen Gaseinlaß 4 und einen Gasauslaß 5, die so angeordnet sind, daß das zu analysierende Gas durch ein dünnes Röhrchen 10 in eine Absorptions- und Reaktionslösung injiziert wird, die in einer kleinen Mulde 6 am Boden des Gefäßes ausgebildet ist. Ein- und Auslaß können, wie dargestellt, waagrecht oder auch derart schräg angeordnet sein, daß das Röhrchen 10 geradlinig zur Mulde 6 verläuft.

Oberhalb dieser Mulde ist das Gefäß mit einem Septum 7 verschlossen, durch das eine Kanüle 8 in das Gefäß eindringen kann. Das Gefäß besteht beispielsweise aus einem Glaskörper, der gegenüber dem zu analysierenden Gas und der Reaktionslösung inert ist. Die Kanüle ist an ein Transfersystem 9 angeschlossen, das hier symbolisch als eine Kolbenspritze dargestellt ist, aber in Wirklichkeit ein automatisches Transfersystem ist, mit dem Reaktionsstoffe und Reinigungsflüssigkeit in das Gefäß dosiert injiziert werden können oder Reaktionsflüssigkeit nach der Exposition entnommen und in eine automatische Flüssigkeitschromatographiesäule überführt werden kann. Das Transfersystem und die Analysesäule sind an sich bekannt und werden beispielsweise unter der Bezeichnung SIL-7A von der Shimadzu Corporation vertrieben. Dort werden viele mit einer Analyseprobe gefüllte Probegläschen mit Hilfe einer automatischen Ablaufsteuerung nacheinander analysiert. Dabei dringt eine Kanüle durch das Septum eines Gläschens, entnimmt eine Probe und transferiert sie in eine Flüssigchromatographiesäule, wo sie getrennt und mit einem geeigneten Detektor nachgewiesen wird. Nach einer Reinigung der Kanüle folgt das nächste Gläschen.

Die erfindungsgemäße Vorrichtung arbeitet vorzugsweise wie folgt: Zuerst entnimmt das Transfersystem über die Kanüle 8 eine bestimmte Menge Waschflüssigkeit aus einem Vorratsgefäß (nicht dargestellt) und füllt diese Waschflüssigkeit in das Reaktionsgefäß 3. Durch Einschalten der Saugpumpe wird die Waschflüssigkeit so im Gefäß verteilt, daß die gesamte Oberfläche bespült wird. Dann wird die Waschflüssigkeit wieder durch die Kanüle 8 aus dem Gefäß abgesaugt und verworfen. Nun ist die Vorrichtung bereit zur Aufnahme einer dosierten Menge einer sauren Lösung von Dinitrophenylhydrazin mithilfe der Kanüle 8, worauf die Entnahme der Luft- oder Gasprobe beginnt. Nach der Probenahme (Exposition), die nach Bedarf mehrere Sekunden, Minuten oder Stunden dauern kann, wird die Zufuhr der zu analysierenden Luft beendet.Als nächster Schritt folgt die vollständige Entnahme der Lösung über die Kanüle 8 und deren Transfer zu einem Dosiersystem (nicht dargestellt) einer Flüssigkeitschromatographiesäule, in der der Schadstoffanteil gemessen wird. Damit ist der Zyklus beendet und ein neuer Zyklus beginnt wie oben mit dem Auswaschen der Kanüle und des Reaktionsgefäßes, dem Einfüllen einer frischen Reaktionslösung in die Mulde 6 und dem erneuten Auswaschen der Kanüle 8.

In die Mulde kann man eine extrem kleine Menge von z.B. 10 bis 100 µl der Reaktionslösung einfüllen, die nach der Exposition insgesamt mit der Kanüle in den Chromatographen transferiert wird. Aufgrund dieser geringen Lösungsmenge, die durch den Wegfall der Leitung zum Chromatographen und den direkten Transfer mit der Kanüle möglich geworden ist, wird die Konzentration des Schadstoffes in der Lösung erhöht und die Reinigung des Gefäßes und der Kanüle deutlich erleichtert und damit der Memory-Effekt soweit verringert, daß reproduzierbare Meßwerte für die Konzentration von z.B. Formaldehyd in der Größenordnung von 1 µg/m³ und auch darunter erhalten werden.

Da der ganze Zyklus ohne menschliche Intervention abläuft, können sehr weitgehende Analysebedürfnisse durch geeignete Programmierung des Transfersystems erfüllt werden. Dies gilt beispielsweise für die Dauer der Expositionszeit und ggf. der Wartezeit zwischen zwei aufeinanderfolgenden Probenentnahmen sowie für die Art und Menge der Reaktions-lösung. Damit kann ein umfangreiches und variables Meßprogramm vorprogrammiert werden und automatisch ohne Einwirkung des Betreibers abgewickelt werden.

Die erfindungsgemäße Vorrichtung kann durch Abwandlung des oben erwähnten bekannten Injektors vom Typ SIL-7A realisiert werden, indem dort lediglich das Gestell für die zahlreichen vorab deponierten Probegläschen entfernt wird und dafür das in der Figur gezeigte Reaktionsgefäß an einer bestimmten Stelle fest eingebaut wird. Das Transfersystem braucht dann nur diese Stelle und die Behälter für die Reinigungsflüssigkeit und die Absorptions- bzw. Reaktionslösung bzw. deren Komponenten anzufahren anstatt der zahlreichen Probegläschen, die bisher in ihrem Gestell nacheinander auf ihre Analyse warteten. Im Rahmen der Erfindung können auch mehr Reaktionsgefäße 3, insbesondere zwei solche Gefäße vorgesehen sein, die abwechselnd verwendet werden, wobei sich eines jeweils in der Expositionsphase befindet und das andere währenddessen für die nächste Expositionsphase vorbereitet wird. Dies erlaubt eine praktisch kontinuierliche Schadstoffüberwachung eines Raumes oder der Außenluft.

Die Erfindung ist nicht auf die im einzelnen beschriebene Vorrichtung und das zugehörige Verfahren beschränkt. So kann man natürlich einen anderen Schadstoff oder andere Schadstoffe als Formaldehyd, z.B. Acetaldehyd, Acrolein und Aceton, mit der erfindungsgemäßen Vorrichtung erfassen, und der Stoff kann sich allgemein in einem anderen Trägergas als Luft befinden. Als Reaktionsstoff dient ggf. auch ein anderer Stoff als Dinitrophenylhydrazin.

## Patentansprüche

1. Verfahren zur automatischen Analyse von flüchtigen Substanzen in Gasen, insbesondere in Luft, das nacheinander folgende Schritte enthält:
a) das zu analysierende Gas wird durch eine Absorptions- und ggf. Reaktions-Lösung geleitet,
b) die Lösung wird mittels einer Chromatographiemethode analysiert,
**dadurch gekennzeichnet, daß** die Absorptions- und ggf. Reaktionslösung sich in einer Mulde am Boden eines Reaktionsgefäßes befindet und nach der Einwirkung durch das zu analysierende Gas vollständig mit einer Kanüle dem Gefäß entnommen und insgesamt der Analyse zugeführt wird.

2. Verfahren nach Anspruch 1, **dadurch gekennzeichnet, daß** als Reaktionsstoff in der Lösung Dinitrophenylhydrazin verwendet wird.

3. Vorrichtung zur Durchführung des Verfahrens nach Anspruch 1 oder 2, **dadurch gekennzeichnet, daß** das Reaktionsgefäß (3) am Boden eine Mulde (6) für die Reaktionslösung besitzt und diesem gegenüber oben mit einem Septum (7) verschlossen ist, durch das die Kanüle (8) bis in die Mulde eindringen kann, daß der Einlaß für das zu analysierende Gas in das Gefäß (3) in einem Röhrchen (10) endet, das in die Mulde mündet, während der mit einer Saugpumpe (2) verbundene Auslaß (5) sich seitlich oberhalb der Mulde befindet, und daß die Kanüle (8) Teil eines an sich bekannten automatischen Transfersystems ist, das außerdem programmgesteuert Waschflüssigkeit in das Gefäß injiziert, diese Flüssigkeit wieder absaugt sowie anschließend frische Lösung bzw. deren Komponenten für die nächstfolgende Analyse in das Reaktionsgefäß dosiert einspeist.

## Claims

1. A method for automatically analysing volatile substances in gases, especially in air, comprising the succession of the following steps:
a) the gas to be analyzed is passed through an absorbent and, as the case may be, a reactive solution,
b) the solution is analyzed by means of a chromatographic method,
**characterized in that** the absorbent and, as the case may be, the reactive solution is injected into a depression at the bottom of a reaction impinger and that after the exposition to the gas to be analyzed this solution is withdrawn completely from the impinger by a cannula and as a whole transferred to the analysis.

2. A method according to claim 1, **characterized in that** the reactive component in the solution is dinitrophenyl -hydrazine.

3. A device for implementing the method according to claim 1 or 2, **characterized in that** the reaction impinger (3) disposes of a depression (6) at the bottom for receiving the reaction solution and is closed at the opposite side by a septum (7) through which the cannula (8) can penetrate into the depression, that the inlet into the impinger for the gas to be analyzed ends up in a pipe (10) which opens into the depression whereas the outlet (5) which is connected to a suction pump (2) is disposed laterally above the depression, and that the cannula (8) is part of an automated transfer system known per se which further injects under the control of a program a rinsing liquid into the impinger, withdraws this liquid thereafter and injects a calibrated quantity of fresh solution or its components into the impinger in view of the next-following analysis.

## Revendications

1. Procédé pour l'analyse automatique de substances volatiles dans un gaz, tel que dans l'air, comportant la succession des étapes suivantes:
a) le gaz à analyser traverse une solution absorbante et le cas échéant réactive;
b) la solution est analysée moyennant une méthode chromatographique,
**caractérisé en ce que** la solution absorbante et, le cas échéant, réactive se trouve dans une dépression du fond d'un récipient de réaction et, après l'exposition au gaz à analyser, elle est extraite complètement du récipient et transferée en total vers l'analyse.

2. Procédé selon la revendication 1, **caractérisé en ce qu'**on utilise du dinitrophényle hydracine en tant que produit de réaction dans la solution.

3. Dispositif pour la mise en oeuvre du procédé selon la revendication 1 ou 2, **caractérisé en ce que** le récipient de réaction (3) présente au fond une dépression (6) pour la solution réactive, et à l'extrémité supérieure il est fermé par un septum (7) par lequel la canule (8) peut pénétrer jusqu'à la dépression, que le canal d'accès par lequel le gaz à analyser est injecté dans le récipient (3) se termine par une tubulure (10) qui débouche dans la dépression, alors que la sortie (5) connectée à une pompe d'aspiration (2), est située au dessus de cette dépression, et que la canule (8) fait partie d'un système automatique de transfert en soi connu, qui, par ailleurs, injecte sous commande d'un programme, dans le récipient un liquide de rinçage, l'élimine plus tard, et fournit au récipient de réaction une quantité dosée de solution réactive fraîche ou de ses composantes en vue de l'analyse suivante.
